(19) Europäisches Patentamt
European Patent Office
Office européen des brevets

(11) **EP 2 263 717 B1**

(12) **EUROPÄISCHE PATENTSCHRIFT**

(45) Veröffentlichungstag und Bekanntmachung des Hinweises auf die Patenterteilung:
**25.07.2018 Patentblatt 2018/30**

(51) Int Cl.:
*A61M 1/36* (2006.01)  *A61M 1/16* (2006.01)

(21) Anmeldenummer: **10010232.6**

(22) Anmeldetag: **10.01.2000**

(54) **Verfahren und Vorrichtung zur Erkennung von Stenosen bei der extrakorporalen Blutbehandlung**

Method and device for recognition of stenoses in extra-corporal blood treatment

Procédé et dispositif destinés à la détection de sténoses pour le traitement sanguin extracorporel

(84) Benannte Vertragsstaaten:
**AT BE CH CY DE DK ES FI FR GB GR IE IT LI LU MC NL PT SE**

(30) Priorität: **14.01.1999 DE 19901078**

(43) Veröffentlichungstag der Anmeldung:
**22.12.2010 Patentblatt 2010/51**

(62) Dokumentnummer(n) der früheren Anmeldung(en) nach Art. 76 EPÜ:
**07010448.4 / 1 829 570**
**00100411.8 / 1 020 199**

(73) Patentinhaber: **Fresenius Medical Care Deutschland GmbH**
**61352 Bad Homburg (DE)**

(72) Erfinder: **Polaschegg, Hans-Dietrich, Dr.**
**9231 Köstenberg (AT)**

(74) Vertreter: **Nordmeyer, Philipp Werner**
**df-mp Dörries Frank-Molnia & Pohlman**
**Patentanwälte Rechtsanwälte PartG mbB**
**Theatinerstraße 16**
**80333 München (DE)**

(56) Entgegenhaltungen:
**WO-A-97/10013      DE-A1- 3 806 248**
**DE-C1- 19 734 002   DE-U1- 9 400 924**
**US-A- 4 174 637**

**Beschreibung**

Die Erfindung betrifft eine Vorrichtung zur Überwachung eines Kreislaufes der extrakorporalen Blutbehandlung auf Stenosen wie in Patentanspruch 1 definiert sowie die in Patentanspruch 2 angegebene Verwendung der Vorrichtung. Die Vorrichtung wertet Druckpulse aus, die entweder durch das Herz oder durch die Blutpumpe des extrakorporalen Kreislaufs erzeugt werden.

In einer Ausgestaltung der Erfindung werden die Druckpulse im extrakorporalen Kreislauf nicht Invasiv, direkt am Schlauch und ohne Verwendung spezieller Druckübertragermembrane gemessen.

Die Erfindung wird in der nachfolgenden Beschreibung und in den Patentansprüchen näher erläutert.

Die extrakorporale Blutbehandlung ist heute ein Standardverfahren, daß vor allem zur Behandlung der Niereninsuffizienz in Form der Hämodialyse, Hämofiltration oder Hämodiafiltration aber auch zur Behandlung der Hypercholesterinämie und von Autoimmunerkrankungen eingesetzt wird. Weitere extrakorporale Behandlungsverfahren basieren auf physikalischen Effekten, z.B. Bestrahlung des Blutes mit Licht, UV Licht oder hochenergetischer Strahlung (z.B. Röntgen, Beta- oder Gammastrahlung). Ferner ist die Anwendung erhöhter oder verringerter Temperatur, magnetischer, elektrischer oder elektromagnetische Felder zur extrakorporalen. Blutbehandlung bekannt

Zur extrakorporalen Blutbehandlung ist ein ausreichend ergiebiger Zugang zum Blutkreislauf des Körpers erforderlich. Bei der chronischen Behandlung muß dieser Zugang über viele Jahre funktionieren. Die Verbesserung der Behandlung hat dazu geführt, daß es viele Langzeitpatienten gibt. Zusätzlich ist die Behandlung älterer Menschen möglich geworden. Beides hat dazu geführt, daß der Blutzugang zunehmend zum Problem wird und deshalb oft die "Achillesferse" der Hämodialyse genannt wird. Als Standard des Blutzuganges hat sich seit vielen Jahren ein unter der Haut liegender shunt zwischen einer Arterie und Vene bewährt. Dieser wird entweder direkt durch die Verbindung zweier Adern (Cimino-Fistel) oder mit Hilfe eines künstlichen Gefäßes (graft) hergestellt. Blutzugänge dieser Art, sowie die Operationstechnik zu ihrer Herstellung sind in allen Standardwerken zur Hämodialyse beschrieben.

Ein häufiges Problem mit diesen Blutzugängen sind Stenosen, das heißt Verengungen der Gefäße, die, wenn sie nicht rechtzeitig erkannt und behoben werden, zum totalen Verschluß und damit Verlust der Fistel bzw. des Graft führen können. Der Einfachheit halber wird weiter unten nur mehr das Wort Fistel verwendet, die Ausführungen betreffen jedoch auch Grafts.

In einer funktionierenden Fistel herrscht ein Blutfluß von typisch 700 ml/min mit einem weiten Bereich von 300 - 1500 ml/min. Auch größere Flüsse wurden gemessen, jedoch neigt man dazu, solche Blutzugänge zu korrigieren, da hohe Blutflüsse zu einer Belastung des Herzens führen.

Im extrakorporalen Blutkreislauf wird Blut meist mit 200 - 500 ml/min gepumpt. Sinkt der Blutfluß in der Fistel unter den Blutfluß im extrakorporalen Kreislauf, so kommt es zur Rezirkulation und damit zu einer Verminderung der Effektivität des Verfahrens. Nun hat man festgestellt, daß es bei Auftreten einer Stenose, die zu einem Blutfluß von weniger als 600 mL/min in Grafts führt, in kurzer Zeit zu einem Totalverschluß kommt. Wird dieser Zustand rechtzeitig erkannt, so kann, durch einen relativ einfachen Eingriff, die Stenose behoben werden, ehe es zu einem Totalverschluß und damit Verlust des Zugangs kommt Da 600 mL/min über dem üblichen Fluß im extrakorporalen Kreislauf liegt, kann dies durch einfache Rezirkulationsmessung nicht erkannt werden. Man ist deshalb bestrebt, den Fistelfluß von Zeit zu Zeit zu messen, um eine Stenose rechtzeitig zu erkennen. Dies ist z.B. mit einem Ultraschall-Dopplerverfahren möglich, wofür aber ein teures Gerät und Spezialisten benötigt werden. Solche Geräte und Kenntnisse sind in einer Dialysestation normalerweise nicht vorhanden. Ferner wurde ein Verfahren entwickelt, bei dem die Blutflußrichtung im extrakorporalen Kreislauf umgekehrt und damit eine Rezirkulation hervorgerufen wird. Der Rezirkulationsanteil wird dann mit einem üblichen Verfahren gemessen und daraus der Fistelfluß errechnet. Auch dieses Verfahren bedarf eines speziellen Gerätes und geschulten Personals. Obwohl es in der Dialysestation durchgeführt werden kann, hat es sich nicht durchgesetzt, da es stets einen Eingriff in die Behandlung erfordert und damit auch nicht kostengünstig ist. Dieses Verfahren ist im US Patent 5685989 beschrieben.

Weiters hat man festgestellt, daß der im extrakorporalen Kreislauf gemessene Druck durch Stenosen in der Fistel beeinfluß wird. Man hat daraus Verfahren abgeleitet, die unter bestimmten Umständen eine Erkennung einer Stenose ermöglichen. In einem Fall hat man empirisch festgestellt, daß bei Verwendung bestimmter Kanülen und bei einem bestimmten Blutfluß, der im extrakorporalen Kreislauf gemessene venöse Rücklaufdruck eine Aussage über eine stromab von der Fistel liegende Stenose ermöglicht. Liegt der venöse Rücklaufdruck über einem bestimmten, zuvor in einer Klinik empirisch bestimmten Grenzwert, so wird auf eine Stenose geschlossen. Eine Beschreibung des Verfahrens ist u.a. in: Schwab SJ Raymond JR Saeed M Newman GE Dennis PA Bollinger RR. Prevention of hemodialysis fistula thrombosis. Early detection of venous stenoses.. Kidney International 1989;36:707-11 publiziert.

Dieses Verfahren hat offensichtliche Nachteile: Zunächst die empirische Bestimmung des Grenzwertes, d.h. man muß entweder Thrombosen in Kauf nehmen oder mit einem Referenzverfahren, z.B. Ultraschall-Dopplerbestimmung vergleichen. Ferner die Verwendung einer bestimmten Kanüle und eines bestimmten Blutflusses für die Messung. D.h. die Behandlung wird zumindest temporär beeinflußt.

Bei einem anderen Verfahren wird der statische Fisteldruck gemessen. Dies erfolgt entweder mit speziellen Drucksensoren in der Höhe der Fistel, wobei diese über eine eigene Kanüle mit der Fistel verbunden werden oder aber im extrakorporalen Kreislauf angebracht werden. Es ist auch bekannt, dazu die bereits im extrakorporalen Kreislauf befindlichen Sensoren einzusetzen. Dabei muß dann die Höhendifferenz zwischen dem Flüssigkeitsniveau im extrakorporalen Kreislauf und der Fistel korrigiert werden. Auch diese Verfahren bedeuten zumindest einen Personalaufwand zur Messung der Niveauunterschiede und Auswertung der Messung. Da der Patient in der Regel die Möglichkeit hat, seine Lage zu verändern, ist eine solche Messung auch nur einmalig möglich, soll der Personalaufwand beschränkt bleiben.

Dieses Verfahren ist in: Besarab A, Al-Saghir F, Alnabhan N, Lubkowski T, Frinak S. Simplified Measurement of Intra-Access Pressure. ASAIO Journal 1996;42:M682-7 beschrieben.

Auch im extrakorporalen Kreislauf kann es zu Stenosen kommen. Dabei handelt es sich um Flußwiderstände in Kanülen, die entweder vom Anwender nicht richtig eingeschätzt werden oder aber um solche, die durch Abknicken oder Abquetschen von Schläuchen oder aber durch Blutgerinnung hervorgerufen werden. Solche Stenosen können entweder zu einer Verringerung des effektiven Blutflusses im extrakorporalen Kreislauf und damit zu einer Verringerung der Effektivität führen oder aber auch zur Hämolyse wegen der hohen Scherkräfte im Bereich der Stenose. Zur Erkennung solcher Stenosen sind im extrakorporalen Kreislauf Drucksensoren, die überwiegend invasiv, d.h. über Druckableitungen oder aber mit speziellen Druckübertragem arbeiten vorgesehen. Diese Sensoren sind aufwendig und fördern die Blutgerinnung. Sie messen den statischen Druck, d.h. im extrakorporalen Kreislauf auftretende Druckpulse werden mit Hilfe von Elektronik-Hardware oder Software geglättet und damit eliminiert. In einem Patent des Erfinders (*Polaschegg HD, inventors. Fresenius AG, assignee. Verfahren zur Funktionsüberwachung (Ausfallerkennung) einer Druckmeßanordnung in einem Flüssigkeitssystem eines medizinischen Gerätes und Vorrichtung zur Durchführung des Verfahrens.* DE patent 3806248. *7/11/91)* werden die Druckpulse zur Erkennung der Funktionsfähigkeit der Druckmeßanordnung herangezogen. Es wird in der DE 3806248 auch darauf hingewiesen, daß damit die Pulsfrequenz des Patienten gemessen werden kann. Ferner wird in der DE 3806248 auch ein Verfahren angegeben um eine Stenose im extrakorporalen Kreislauf zwischen der Blutpumpe und dem venösen Drucksensor über eine Phasenverschiebung zu erkennen.

Aus der WO 97/10013 A1 ist eine Vorrichtung zum Detektieren des Zustands eines Blutgefäßzugangs bekannt, bei der die Abwesenheit eines Pulssignals eine Fehlfunktion im Blutgefäßzugang anzeigt.

Die US 4,174,367 A offenbart ein Drucküberwachungssystem, das zur Verwendung mit extrakorporalen Kreislaufsystemen geeignet ist, und Drucksensoren einsetzt, die auf Dehnmessstreifen basieren.

Die DE 94 00 924 Ul beschreibt einen extrakorporalen Blutkreislauf für ein Dialysegerät mit vier Kreislaufabschnitten, in dem der arterielle Druck und der venöse Druck mit Druckmessern überwacht werden.

Die vorliegende Erfindung hat die Aufgabe eine Vorrichtung anzugeben, mit der eine Früherkennung von Stenosen im Bereich der Fistel, aber auch die kontinuierliche Überwachung des extrakorporalen Kreislaufs möglich ist. Zusätzlich kann mit dieser Vorrichtung auch noch in bekannter Weise die Drehzahl der Blutpumpe und der Pulsschlag des Patienten bestimmt werden. Die Vorrichtung nutzt die Messung der Amplitude, gegebenenfalls korrigiert mit einer Funktion der Frequenz, der im extrakorporalen Kreislauf bei laufender bzw. stehender Blutpumpe gemessenen Druckpulse. Bei Untersuchungen in der Klinik wurde festgestellt, daß stromab des Blutzugangs bei stehender Blutpumpe der Pulsschlag des Patienten im extrakorporalen Kreislauf gemessen werden kann. Allerdings ist dies mit den üblichen, in Dialysegeräten eingebauten Druckmeßeinrichtungen nicht möglich, da diese, wie beschrieben, künstlich gedämpft werden Die Beobachtung wurde vielmehr mit ungedämpften Drucksensoren gemacht. Die Drucksensoren herkömmlicher Dialysegeräte wären allerdings durchaus in der Lage solche Druckpulse zu erfassen, sofern die elektronische Dämpfung entfernt wird.

Überraschenderweise wurde festgestellt, daß die Amplituden dieser Pulse bei Vorliegen einer Stenose höher sind. Schließlich wurde festgestellt, daß die vom Pulsschlag herrührenden Pulse auch noch bei laufender Blutpumpe zu erkennen sind, wenn eine Stenose vorliegt. Bei Betrieb eines extrakorporalen Kreislaufes konnte zunächst kein Zusammenhang zwischen den gemessenen Drucken vor der Blutpumpe (p(art) bzw. in der venösen Tropfkammer (p(ven)) und den von der Blutpumpe erzeugten Druckpulsen festgestellt werden. Überraschenderweise wurde dann aber festgestellt, daß ein annähernd linearer Zusammenhang zwischen den Amplituden der Druckpulse und dem Mitteldruck festgestellt werden kann, wenn die Compliance des Systems durch Entfernung der Luft aus dem System verringert wird.

Wie bereits erwähnt, werden zur Drucküberwachung im extrakorporalen Kreislauf entweder Systeme verwendet, die über eine .teilweise mit Luft gefüllte Druckableitung verfügen oder aber solche, die spezielle Druckübertragungsmembrane verwenden. Man hat auch versucht, den Druck direkt am Schlauch zu messen, d.h. die Schlauchwand zur Druckübertragung zu verwenden. Dies ist bei den überwiegend aus PVC hergestellten Schläuchen hauptsächlich an deren Kriechverhalten gescheitert. Deshalb wurde in der DE4106444 ein Verfahren angegeben, um die Auswirkung des Kriechverhaltens durch vorgespannte Lagerung des Schlauches zu verringern (*Steinbach B, Flaig H-J, inventors. Fresenius AG, assignee. Verfahren und Vorrichtung zur Messung eines Innendrucks in einem Schlauch.* DE patent 4106444. *07/23/92).* Nun hat sich überraschenderweise herausgestellt, daß die Druckpulse im interessierenden Frequenzbereich mit Hilfe eines "acoustic contact sensor" der Fa. Apollo Research Corp. Depew, NY, USAgemessen werden können. Es handelt sich dabei um einen Sensor in Form eines flachen Zylinders, der direkt auf den Schlauch aufgepreßt wird, wobei dieser nur geringfügig verformt wird.

Um den durch die Blutpumpe im extrakorporalen Kreislauf erzeugbaren Druck zu begrenzen, werden peristaltische Blutpumpen gewöhnlich so ausgelegt, daß die Okklusion der Rollen oberhalb eines bestimmten Druckes, typischerweise etwa 1 - 2 bar verringert und damit der Druck begrenzt wird. Es kommt dabei zu einem zeitweisen Zurückströmen der Flüssigkeit durch den teilokkludierten Schlauch in den Ansaugbereich. Wird Blut gepumpt, so wird dieses durch die dabei auftretenden Scherkräfte hämolysiert, was zu lebensbedrohlichen Komplikationen führen kann. Überraschenderweise wurde festgestellt, daß dieser Zustand durch eine Zunahme der Pulsamplituden sowohl stromab der Blutpumpe als auch stromauf der Blutpumpe festgestellt werden kann obwohl stromab der Mitteldruck wegen der Flußbegrenzung nicht weiter zunimmt und stromauf (auf der Ansaugseite) sogar eine Verringerung des mittleren Ansaugdruckes gemessen wird.

Die Erfindung wird nun an Hand von Abbildungen und Beispielen näher erläutert:

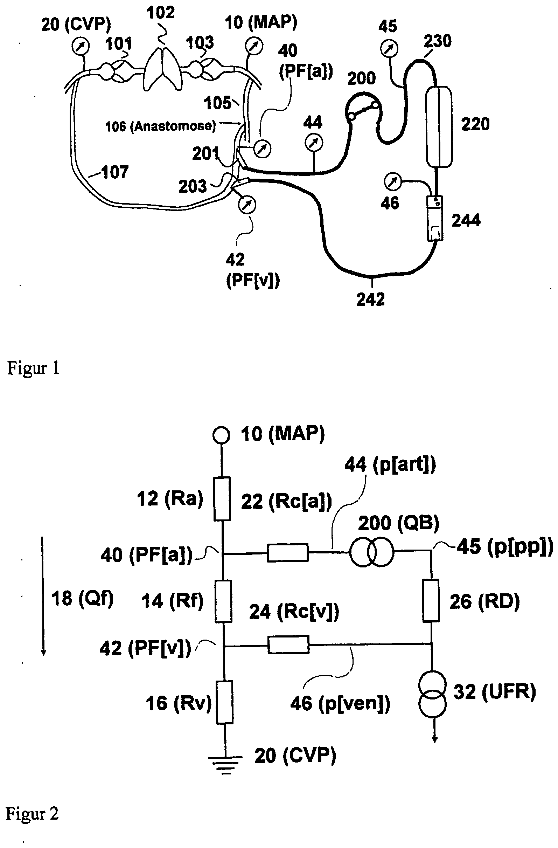

Es zeigt Figur 1 symbolisch den systemischen Blutkreislauf des Menschen mit einem angeschlossenen extrakorporalen Kreislauf.

Figur 2 zeigt einen hydraulischen Ersatzkreislauf für den systemischen und extrakorporalen Kreislauf zur Darstellung von Fließwiderstand und Druck.

Figur 3 stellt qualitativ die Veränderung des arteriellen und venösen Fisteldrucks bei Auftreten einer Stenose in Abhängigkeit vom Bereich der Stenose dar.

Figur 4 zeigt systemische Druckpulse, gemessen mit einem Drucksensor hoher Zeitauflösung im arteriellen Schenkel des extrakorporalen Kreislaufs

Figur 5 zeigt systemische Druckpulse, gemessen mit einem Drucksensor hoher Zeitauflösung im arteriellen Schenkel des extrakorporalen Kreislaufs bei vorliegen einer Stenose in der Fistel.

Figur 6 zeigt systemische Druckpulse überlagert von Druckpulsen der Blutpumpe.

Figur 7 zeigt den Zusammenhang zwischen geglättetem arteriellen Fisteldruck und der am selben Ort gemessenen Pulsamplitude gemessen an mehreren Patienten.

Figur 8 zeigt die Abhängigkeit der vor der Blutpumpe gemessenen Druckpulsamplitude von der Pumpgeschwindigkeit

Figur 9 zeigt den Druck stromab der Blutpumpe p(pp) und an der venösen Meßstelle p(ven) über einen Zeitraum innerhalb dessen der Schlauchquerschnitt zwischen den beiden Sensoren so stark verringert wurde, daß die Okklusion der Blutpumpe durch den-

Gegendruck teilweise aufgehoben wurde.

Figur 10 zeigt den Druck vor der Blutpumpe p(art) und an der venösen Meßstelle p(ven). Vorgang wie unter Figur 9 beschrieben.

Figur 11 zeigt die Anordnung eines Drucksensors direkt am Schlauch.

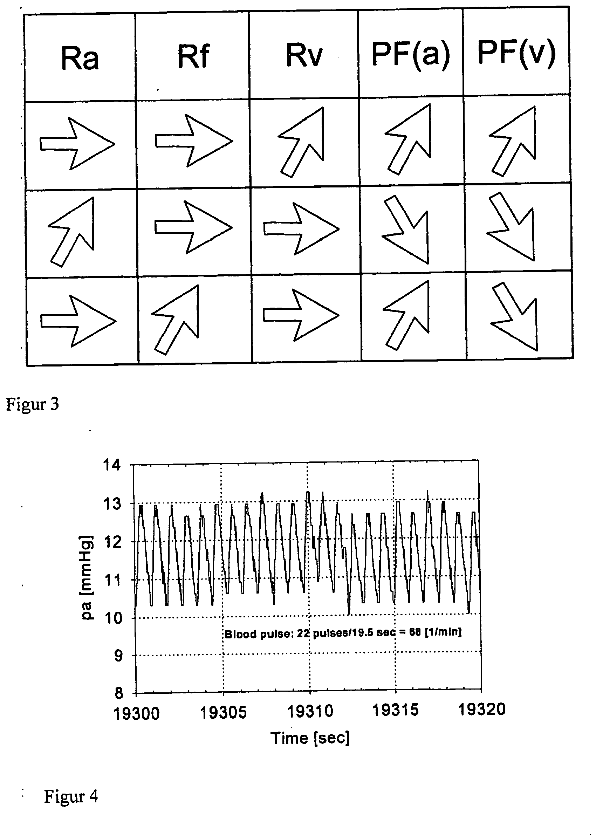

Figur 1 zeigt symbolisch den systemischen Kreislauf des Menschen und einen angeschlossenen extrakorporalen Kreislauf. Blut wird vom rechten Herzen 101 durch die Lunge 102 zum linken Herzen 103 gepumpt und gelangt von dort in das nicht näher bezeichnete arterielle System, in dem der Druck 10 (MAP, mittlerer arterieller Blutdruck) herrscht. Von dort gelangt Blut in eine periphere Armarterie (105) die mit Hilfe einer Anastomose 106 mit einer peripheren Vene 107 verbunden ist. Diese transportiert Blut zur Vena Cava (nicht näher bezeichnet) in der der Zeritralvenöse Druck (CVP) 20 herrscht. Von dort gelangt das Blut wieder zum rechten Herzen womit der systemische Blutkreislauf geschlossen ist. An der Verbindung zwischen Arterie und Vene (106) wird durch den arteriellen Druck die Vene aufgeweitet und es bildet sich eine punktierbare Fistel. In diese Fistel wird eine arterielle Blutzugangskanüle (201) eingestochen. Von dort gelangt das Blut über ein arterielles Schlauchsystem (230) zunächst zu einer peristaltischen Blutpumpe (200), die das Blut durch den extrakorporalen Kreislauf pumpt und weiter zur extrakorporalen Behandlungseinheit (z.B. Hämodialysator, Hämofilter, Hämodiafilter, Plasmafilter, Hämoadsorber). Vom Dialysator gelangt das Blut zurück über das venöse Schlauchsystem 242, in dem üblicherweise eine venöse Tropfkammer 244 eingefügt ist, zur venösen Kanüle 203 und zurück zum systemischen Kreislauf. Im systemischen Kreislauf herrscht an der arteriellen Punktionsstelle der arterielle Fisteldruck PF(a) 40 und an der venösen Punktionsstelle der venöse Fisteldruck PF(v) 42. Im extrakorporalen Kreislauf herrscht vor der Pumpe der arterielle Druck p(art) der mit einem Sensor 44 gemessen wird. Stromab der Pumpe 200 wird vor dem Dialysator häufig auch noch mit einem weiteren Drucksensor (45) der Druck pp (postpump arterial pressure) gemessen. Stromab des Dialysators 220 wird im venösen Schlauchsystem der venöse Rücklaufdruck p(ven) mit Hilfe eines Sensors 46 gemessen, der üblicherweise mit einer nicht näher bezeichneten Druckableitung mit der venösen Tropfkammer verbunden ist. Die Drucksensoren 44,45 und 46 bzw. die damit verbundenen Auswerteelektroniken sind üblicherweise so ausgelegt, daß Pulsationen der Blutpumpe geglättet werden. Die Glättungszeitkonstante liegt dabei in der Größenordnung einiger Sekunden. Figur 2 zeigt das Ersatzschaltbild. Systemisch fließt das Blut vom zentralen arteriellen System (10), das unter dem mitteleren arteriellen Druck steht (MAP) zum zentralen venösen System (20) in dem der zentrale Venendruck (CVP) herrscht. Das Druckgefälle teilt sich entsprechend den Strömungswiderständen Ra(12), Rf(14) und Rv(16) auf. Dabei ist Ra(12) der arteriell Widerstand der den Strömungswiderstand der Anastomose beinhaltet, Rf(14) ist der Widerstand der Fistel zwischen arteriellem und venösem Blutzugang, Rv(16) ist der venöse Strömungswiderstand. Für den Fall eines künstlichen Adernersatzes (graft) zwischen Arterie und Vene beinhaltet dieser Strömungswiderstand auch den der venösen Anastomose. Wenn der extrakorporale Kreislauf nicht betrieben wird, so fließt in diesem Kreis der Blutstrom Qf(18). Im extrakorporalen Kreis erzeugt die Blutpumpe 200 den Fluß QB. Symbolisch dargestellt ist ferner eine Ultrafiltrationspumpe (32), die den Fluß UFR im Dialysator entzieht. Der arterielle Strömungswiderstand 22 und der venöse Strömungswiderstand 24 werden hauptsächlich durch den Strömungswiderstand der Kanülen bestimmt. Sie sind annähernd gleich groß. Der Strömungswiderstand des Dialysators ist mit 26 (RD) symbolisiert. Zwischen Blutpumpe (200) und Dialysator (220, Fig1) mit Strömungswiderstand RG(26) ist häufig noch ein Drucksensor pp (45) eingefügt, der die Aufgabe hat, Erhöhungen des Strömungswiderstandes zwischen Blutpumpe (200) und venöser Druckmeßstelle (46) zu erkennen. Eine Erhöhung des Strömungswiderstandes durch Abknicken des Schlauches hat in der Vergangenheit bereits zu Hämolyse mit tödlichem Ausgang geführt. Die Drucke im Blutzugang sowie im extrakorporalen Kreislauf sind symbolisch mit den gleichen Bezugszeichen wie in Figur 1 gekennzeichnet. Durch Anwendung des Ohm'schen Gesetzes, das auf die Hydraulik übertragbar ist, können die Drucke im Kreislauf bei bekannten Widerständen unschwer errechnet werden. Es ist einsichtig, daß der Druck Pf(v) (42) ansteigen wird, wenn der Widerstand Rv (16) ansteigt, was gleichbedeutend mit einer Stenose ist. Stenosen können nun im arteriellen oder venösen Bereich als auch im Bereich der Fistel zwischen den Blutzugängen auftreten. Aus der qualitativen Analyse der beiden Drucke Pf(a) (40) und pf(v) (42) können nun folgende Schlüsse gezogen werden, die in Figur 3 graphisch dargestellt sind: Eine Stenose im venösen Bereich (Erhöhung von RV) bewirkt sowohl einen Anstieg des arteriellen (PF(a)) als auch des venösen (PF(v) Fisteldrucks. Eine arterielle Stenose bewirkt ein Absinken beider Drucke und eine Stenose in der Fistel bewirkt einen Anstieg des arteriellen und ein Absenken des venösen Drucks.

Stenosen (Einengung des Strömungsquerschnitts) können aber auch im extrakorporalen Kreislauf auftreten. Auch sie können durch Veränderung der Drucke p(art), p(pp) und p(ven) erkannt werden.

Im Prinzip lassen sich die systemischen Drucke PF(a)(40) und PF(v)(42) mit Hilfe der im extrakorporalen Kreislauf angebrachten Drucksensoren (44) und (46) messen. Dazu ist es jedoch notwendig, die hydrostatischen Druckunterschiede, bedingt durch die Höhendifferenzen zwischen den Flüssigkeitssäulen an den Meßorten und an der Fistel zu berücksichtigen. Eine solche Messung muß nun jedesmal durchgeführt werden, wenn die Drucke bestimmt werden sollen. Wie Eingangs beschrieben ist dies aufwendig. Zweckmäßigerweise sollte es zu Beginn der Dialyse durchgeführt werden. Vom Betriebsablauf ist dies aber der Zeitpunkt, da das Pflegepersonal ohnehin voll ausgelastet ist.

Nun hat sich durch Beobachtungen in der Klinik überraschend herausgestellt, daß sich bei stehender Blutpumpe der Pulsschlag des Patienten im extrakorporalen Kreislauf beobachten läßt, wenn die bei Dialysegeräten übliche Dämpfung der Drucksensoren unterbleibt. Es hat sich ferner überraschend herausgestellt, daß die Höhe dieser Druckpulse dem mittleren Druck an der Meßstelle proportional ist. Es wurde erkannt, daß das Ersatzschaltbild auch für pulsierenden Fluß gilt(vergleichbar mit Wechselstrom) wenn die Zeitkonstante des Kreises klein gegen die Frequenz ist. Die Zeitkonstante des Kreises ist das Produkt aus Strömungswiderstand und Nachgiebigkeit der Gefäße (Compliance). Im elektrischen Analogon entspricht dies dem Produkt aus Widerstand und Kapazität R*C. Das weiter Überraschende an den Meßergebnissen ist, daß die Zeitkonstante des systemischen Kreises offensichtlich vernachlässigt werden kann. Es hat sich dann weiter herausgestellt, daß die systemischen Pulse im extrakorporalen Kreislauf auch bei laufender Blutpumpe beobachtet werden können. Ohne besondere Auswerteverfahren ist dies allerdings nur bei kleiner Blutpumpengeschwindigkeit möglich. In einer Ausgestaltung der Erfindung ist jedoch vorgesehen, den Druckverlauf mit einem geeigneten Auswerteverfahren, z.B., der Fourieranalyse nach Frequenzen zu trennen und damit den Pulsschlag nach Frequenz und Amplitude von der bekannten Frequenz der Blutpumpe zu trennen.

Figur 4 zeigt den Druckverlauf ohne statische Druckkorrektur, gemessen an Sensor 44 (p(art)) mit einer Zeitauflösung von 1/20 [sec] am Ende einer Dialysebehandlung. Es handelt sich um eine "normale" Fistel mit einem typischen Fisteldruck von etwa 25 mmHg. Aus der Zahl der Pulse pro Zeiteinheit läßt sich der Pulsschlag des Patienten errechnen. Die Amplitude der Pulse beträgt etwa 2.5 mmHg (peak-peak) bei einem unkorrigierten Mitteldruck von etwa 11.7 mmHg.

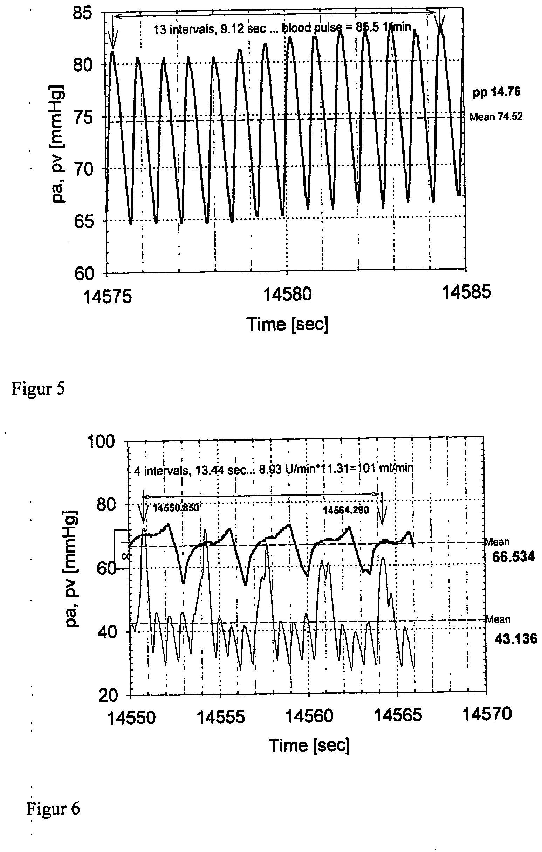

Figur 5 zeigt einen Druckverlauf analog Figur 4, gemessen jedoch an einer Fistel mit einer Stenose zwischen arteriellen und venösem Anschluß und somit erhöhtem Widerstand Rf (14). Wie man erkennt, sind sowohl der unkorrigierte Mitteldruck (74.5 mmHg) als auch die Pulsamplitude (14.8 mmHg) wesentlich höher. Wiederum läßt sich aus der Zahl der Druckpulse pro Zeiteinheit der Pulsschlag des Patienten errechnen.

Figur 6 zeigt die arteriellen und venösen Druckpulse bei einem Blutfluß von -100 mL/min im extrakorporalen Kreislauf. Die Daten wurden unmittelbar vor den Daten aus Figur 4 aufgenommen. Die untere Linie stellt die arteriellen Druckpulse dar. Die hohen Pulse stammen von der Blutpumpe während die kleinen dazwischen vom Pulsschlag des Patienten stammen. Aus der Zahl der von der Blutpumpe erzeugten Pulse pro Zeiteinheit läßt sich die Zahl der Umdrehungen der Blutpumpe pro Zeiteinheit errechnen. Damit wiederum mit einer bekannten Geometriekonstante die Pumprate. Die Zahl der Pulse pro Umdrehung wird durch die Zahl der Rollen bei einer peristaltischen Blutpumpe bestimmt. Bei n(2) Rollen sind es n(2) pro Umdrehung.

Die obere Kurve von Figur 6 zeigt den Druckverlauf am Sensor 46 (p(ven)). Aus ihnen läßt sich ebenfalls die Blutpumpenrate errechnen. Ferner erkennt man, daß sie gegenüber den arteriellen Pulsen phasenverschoben sind. Systemische Druckpulse des Patienten sind nicht zu erkennen. Das liegt daran, daß es auf Grund der Compliance der teilweise luftgefüllten Tropfkammer (244) zu einer starken Dämpfung kommt und die venösen Pulse bereits am Ort 42 (PF(v)) wegen des erhöhten Strömungswiderstandes 14 (Rf) bei diesem Patienten gering sind.

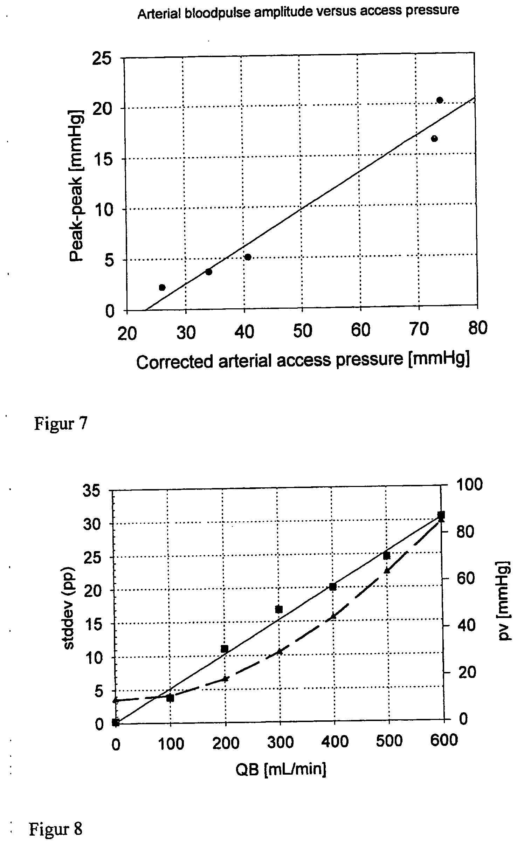

Figur 7 zeigt nun die Auswertung von Druckpulsen an einigen Patienten. Aufgetragen ist die Amplitude (peak-peak) der Druckpulse gemessen mit Sensor 44(p(art)) im extrakorporalen Kreislauf bei stehender Blutpumpe 200 gegen den auf statische Einflüsse korrigierten mittleren arteriellen Fisteldruck PF(a) (40), Wie man sieht besteht gute Linearität.

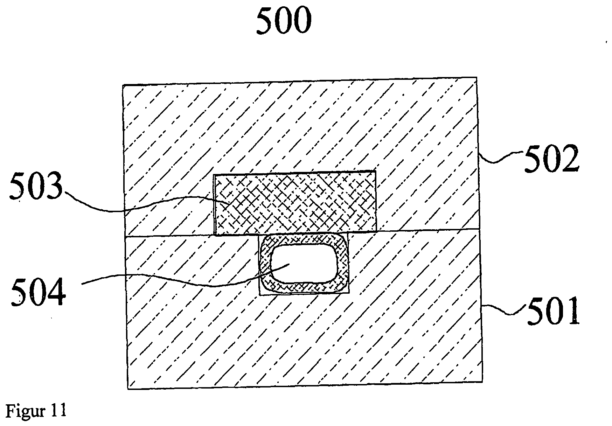

Figur 8 zeigt den gemittelten Druck gemessen an 46 (p[ven], Dreiecke, strichlierte Kurve) sowie die Standardabweichung der Druckpulse gemessen an 44 (p[art], Quadrate, durchgezogene Kurve). Die arteriellen Druckpulse (46) wurden dabei mit einem Druckaufnehmer gemessen, der direkt am Schlauch angepreßt wurde (Figur 11) um jegliche Compliance von einer Druckableitung zu vermeiden. Bei Vorliegen einer Compliance kommt es nämlich zu einer stark frequenzabhängigen Dämpfung des Signals. Der Druck 46 (p [ven]) wurde zum Vergleich angegeben.

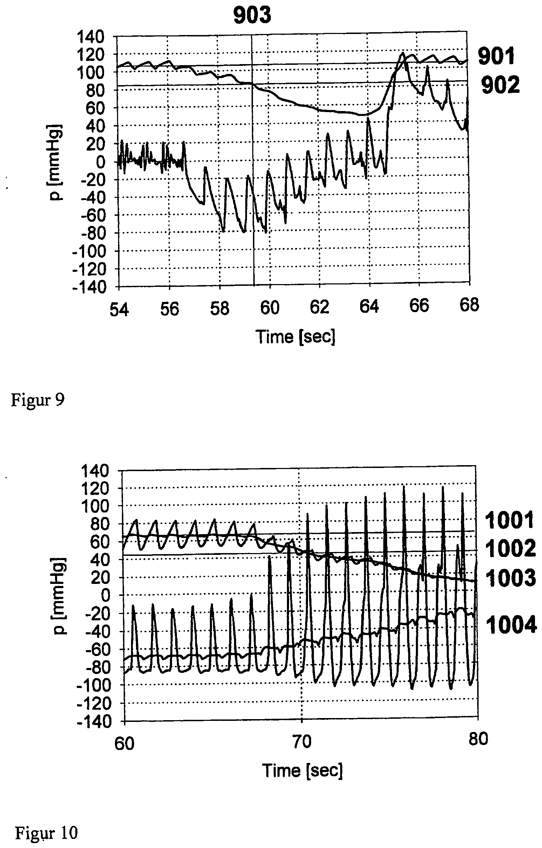

Figur 9 zeigt den zeitlichen Verlauf des Druckes gemessen am Ort 46 (p[ven], obere Kurve) und am Ort 45 (p[pp]) stromab der Blutpumpe (untere Kurve). Ferner sind als waagrechte Linien dargestellt der mittlere venöse Druck (46, p[ven]) als 901 und 20 mmHg darunter eine fiktive Alarmgrenze (902). Bei einem üblichen Dialysegerät würde ein Alarm ausgelöst werden, wenn diese Alarmgrenze unterschritten würde. Dies wäre zum Zeitpunkt 903 der Fall. Der Versuch wurde in-vitro mit Wasser von 37°C und dem in Figur 1 dargestellten extrakorporalen Kreislauf durchgeführt. Der Patient wurde durch ein thermostatisiertes Gefäß ersetzt, der Dialysator durch ein Kurzschlußstück. Kanülen 16g/25 mm wurden verwendet. Der Fluß betrug 400 mL/min.

Zur Aufnahme dieser Kurve wurde der Blutschlauch stromab der Pumpe und des Sensors 45 (p[pp]) langsam abgeklemmt wodurch es zu einem Anstieg des Mitteldrucks stromab der Pumpe kommt, der schließlich zu einer zeitweisen Aufhebung der Okklusion der Pumpe und zu einem Rückgang des Pumpenflusses führt. Schon bei einer kurzzeitigen Aufhebung der Okklusion und ehe noch ein Abfall des Flusses den venösen Druck auf den Wert 902 reduziert, kommt es zu einem deutlichen Ansteigen der Druckpulsamplitude gemessen an 45. Messung der Druckpulse stromab der Blutpumpe erlaubt somit die empfindliche Erkennung des Okklusionsgrenzwertes bei dem es zur Hämolyse von Blut kommt. Der herkömmliche venöse Drucksensor würde erst bei einem Druckabfall von ca. 10% ansprechen. Dabei würden allerdings bereits 10% des Blutflusses hämolysiert werden. Der in Figur 9 dargestellte Versuch stellt die Vorgänge der Praxis im Zeitraffer dar. In der Praxis ist es durchaus möglich, daß der extrakorporale Kreislauf mit einer Teilokklusion über längere Zeit betrieben wird. Selbst wenn Alarm ausgelöst wird, so ist die wahrscheinlichste Reaktion des Personals, die Alarmgrenzen zu verstellen, da der venöse Druckmonitor nicht eindeutig gefährliche Situationen anzeigt. Die vorliegende Erfindung erlaubt nun eine eindeutige Anzeige des gefährlichen Zustandes.

Figur 10 stellt einen weiteren Versuch mit der zu Figur 9 beschriebenen Anordnung dar, jedoch wurde die Pumpe mit 300 mL/min betrieben. Die Kurve enthält wieder waagrechte Linien 1001 und 1002 für den venösen Mitteldruck zu Beginn und den darunterliegenden, fiktiven Grenzwert. Ferner den venösen Druck (p[ven], 46) gemessen mit hoher Zeitauflösung und gemittelt (obere Kurven, 1003) und den vor der Pumpe, mit einem herkömmlichen Druckaufnehmer, der über eine luftgefüllte Druckmeßleitung angeschlossen ist gemessenen Druck p[art], 44 in hoher Zeitauflösung sowie, geglättet (1004). Wie man erkennt steigt die Druckpulsamplitude auf der arteriellen Seite stark an, wenn die Okklusion vermindert wird. Der mittlere Druck wird dabei absolut sogar geringer (weniger negativ), da der mittlere Fluß abnimmt. Man kann den unter Figur 9 beschriebenen gefährlichen Zustand daher sogar mit der konventionellen Meßanordnung erkennen in dem man den Quotienten aus der Druckpulsamplitude und dem mittleren Druck überwacht, der im Normalzustand annähernd durch eine Konstante (Widerstand der Kanüle, Compliance der Druckableitung, Frequenz der Pumpe) beschrieben werden kann. Alternativ kann bei gleichbleibender Pumprate die Druckpulsamplitude auf größere Abweichung überwacht werden (>50%).

Figur 11 stellt die Meßanordnung mit dem, direkt am Schlauch, die Druckpulse messenden Sensor dar. Es ist 500 die Halterung für den Sensor 503, die in zwei Teile 501 und 502 zerlegbar ist, damit der Schlauch 504 eingelegt werden kann. Der Schlauch 504 wird beim Zusammenbau der Halterung leicht verformt, so daß eine mecahnische Verbindung zum Sensor 503 hergestellt wird. Beim beschriebenen Versuch wurde der Schlauchdurchmesser (nominal 6mm) in Richtung der Anpressung um 1 mm vermindert. Der Sensor ist elektrisch mit einer Auswerteeinheit verbunden, die nicht näher dargestellt ist. Solche Auswerteeinheiten und Meßprogramme sind am Markt erhältlich.

Die Messung der Druckpulse kann mit den in den Dialysegeräten eingebauten Drucksensoren erfolgen. Ferner mit vom Dialysegerät unabhängigen Drucksensoren, die entweder über eine teilweise luftgefüllte Verbindung oder eine Membrane an den extrakorporalen Kreislauf angeschlossen werden. Bevorzugt ist die Messung über eine Sensoranordnung, bei der der Sensor direkt auf den Schlauch aufgepreßt wird, möglich. Die Auswertung der Druckpulse kann entweder durch Bestimmung der Pulsamplitude (peak-peak) erfolgen, wobei das Resultat über eine vorbestimmbaren Zeitraum gemittelt werden kann (z.B. 3 sec) um Artefakte von z.B. Patientenbewegungen zu unterdrücken. Eine bevorzugte Methode ist die Berechnung der Standardabweichung über einen vorgebbaren Zeitraum (z.B. 3 sec), da es dafür einfache Softwareroutinen gibt. Selbstverständlich ist die Verwendung anderer Algorithmen, die ein Maß für die Pulsschwankungen liefern, möglich. Auch ist es möglich, zur Steigerung der Empfindlichkeit bestimmte Frequenzen oder Frequenzbereiche zu verstärken, abzuschwächen oder ganz allgemein einer Filterkurve zu unterwerfen.

Zur Korrektur der Meßwerte kann auch das Signal der Blutpumpe herangezogen werden. Bei Vorliegen einer Compliance kann es zu einer frequenzabhängigen (=blutpumpengeschwindigkeitsabhängigen) Dämpfung des Druckpulssignals kommen. In diesem Fall kann das gemessene Signal entsprechend einer Funktion

$$A(korr) = A * f(QB)$$

korrigiert werden, bevor es der abschließenden Auswertung unterworfen wird. A(korr) ist dabei das korrigierte Amplitudensignal, das der Amplitude, gemessen ohne Compliance, entspricht. A ist das gemessene Signal und f(QB) eine für eine bestimmte Anordnung zuvor zu bestimmende Funktion. Die Korrektur kann direkt am Meßsignal oder aber am abgeleiteten Signal (z.B. Standardabweichung) erfolgen.

Wie Eingangs erwähnt, ist die Erfindung für alle extrakorporalen Blutbehandlungseinrichtungen geeignet. Anstelle von Kanülen können dabei Katheter zum Einsatz kommen. Anstelle von Dialysatoren die entsprechenden Blutbehandlungseinrichtungen, z.B. Hämoperfusionspatronen, Oxygenatoren, Wärmetauscher, Kartuschen zur Bestrahlung mit elektromagnetischer Strahlung oder zur Behandlung mit elektromagnetischen Feldern. Sind mehrere solcher Einrichtungen in Serie angeordnet, so kann vorteilhafterweise ein weiterer Sensor zwischen den Behandlungseinrichtungen vorgesehen werden.

[0039] - Die Erkennung von Stenosen in einem Blutzugang, der durch einen shunt zwischen einer Arterie und Vene gebildet wird (graft, Fistel) kann durch Einbeziehung des systemischen Blutdrucks verbessert werden. Wie aus dem Ersatzschaltbild in Figur 2 erkennbar ist, errechnen sich die relativen Widerstände aus dem Verhältnis des gesamten Druckabfalls (MAP-CVP) zum Druckwert der am Blutzugang gemessen wird (PF(v), PF(a)). Der zentralvenöse Druck kann vernachlässigt werden, so daß das Verhältnis PF/MAP gebildet werden kann. Nun ist der mittlere arterielle Druck eine Funktion des systolischen und diastolischen Drucks. Dennoch kann es bei der Auswertung der Druckpulse am Blutzugang von Vorteil sein, nicht mit dem MAP sondern mit der Differenz aus systolischem und diastolischem Druck zu normieren. Die Grenzwerte, bei denen auf eine Stenose in einem bestimmten Bereich geschlossen wird hängen von der Art des Blutzugangs ab. Bei grafts, gemessen am arteriellen Blutzugang wird für den statischen Druck ein Normalwert von 53% des mittleren arteriellen Blutdrucks (MAP) angegeben (Besarab A, Frinak S. The prevention of access failure: pressure monitoring.. ASAIO Journal 1998;44:35-7). Bei der Auswertung der Druckpulse entspricht dies etwa der mit der Differenz aus systolischen und diastolischen Druck normierten Pulsamplitude. Zwischen MAP, systolischen und diastolischen Druck sowie zwischen Pulsamplituden und Standardabweichung bestehen bekannte mathematische Verhältnisse, so daß eine Umrechnung jederzeit erfolgen kann.

Letztlich wird die Festlegung der Grenzwerte der klinische Praxis angepasst werden müssen. Eine erfindungsgemäße Auswerteinheit wird mindestens den Wert der Pulsamplitude eines Druckes angeben. Da gewöhnlich Angaben zu den systemischen Drucken vorliegen kann in einer weiteren Ausgestaltung der normierte Druck errechnet und ausgegeben werden. Schließlich sind auch noch Grenzwerte vorgebbar, bei deren Unter- bzw. Überschreiten eine Warnung angezeigt werden kann.

Zusammenfassend können mit der vorliegenden Erfindung ohne zusätzliche, kostspielige Hardware bzw. mit vereinfachter Hardware Stenosen bei der extrakorporalen Blutbehandlung erkannt werden, die bisher entweder nicht oder nur mit erheblich höherem Aufwand festgestellt werden konnten. Dadurch kann die Sicherheit und Zuverläsigkeit der extrakorporalen Blutbehandlung erhöht werden.

**Patentansprüche**

1. Vorrichtung zur Überwachung eines Kreislaufes der extrakorporalen Blutbehandlung auf Stenosen, **gekennzeichnet durch** wenigstens einen Drucksensor, der im Bereich zwischen der Blutpumpe und der Blutbehandlungseinheit angeordnet ist, und der Druckpulse im extrakorporalen Kreislauf misst, und eine damit verbundene Auswerteinheit, welche mindestens den Wert der Pulsamplitude dieser Druckpulse angibt, und bei der Grenzwerte vorgebbar sind, bei deren Unter- bzw. Überschreiten eine Warnung angezeigt werden kann.

2. Verwendung einer Vorrichtung zur Überwachung von Stenosen in einem extrakorporalen Blutbehandlungskreislauf nach Anspruch 1, **dadurch gekenn-**

**zeichnet, dass** mit wenigstens einem Drucksensor, der im Bereich zwischen der Blutpumpe und der Blutbehandlungseinheit angeordnet ist, Druckpulse im extrakorporalen Kreislauf gemessen werden und mit einer damit verbundenen Auswerteeinheit mindestens der Wert der Pulsamplitude dieser Druckpulse angegeben wird, wobei bei der Auswerteeinheit Grenzwerte vorgebbar sind, bei deren Unter- bzw. Überschreiten eine Warnung angezeigt werden kann.

## Claims

1. Apparatus for monitoring a circuit of the extracorporeal blood treatment system for stenoses, **characterised by** at least one pressure sensor which is arranged in the region between the blood pump and the blood treatment unit and which measures pressure pulses in the extracorporeal circuit, and an associated evaluation unit which indicates at least the value of the pulse amplitude of these pressure pulses and in which limit values can be preset, it being possible for a warning to be displayed when the value falls below or exceeds said limit values.

2. Use of an apparatus for monitoring stenoses in an extracorporeal blood treatment circuit according to claim 1, **characterised in that** pressure pulses in the extracorporeal circuit are measured with at least one pressure sensor which is arranged in the region between the blood pump and the blood treatment unit, and at least the value of the pulse amplitude of these pressure pulses is indicated with an associated evaluation unit, wherein limit values can be preset in the evaluation unit, it being possible for a warning to be displayed when the value falls below or exceeds said limit values.

## Revendications

1. Dispositif pour le contrôle d'un circuit de traitement extracorporel du sang en matière de sténose, **caractérisé par** au moins un capteur de pression, qui est agencé dans la zone entre la pompe sanguine et l'unité de traitement du sang et qui mesure des impulsions de pression dans le circuit extracorporel, et par une unité d'exploitation reliée à celui-ci, qui donne au moins la valeur de l'amplitude d'impulsion de ces impulsions de pression et par laquelle des valeurs limites peuvent être prescrites, un avertissement pouvant être affiché lorsque lesdites valeurs limites sont dépassées par une valeur inférieure ou supérieure.

2. Utilisation d'un dispositif pour le contrôle de sténoses dans un circuit de traitement extracorporel du sang selon la revendication 1, **caractérisé en ce que** des impulsions de pression dans le circuit extracorporel sont mesurées avec au moins un capteur de pression qui est agencé dans la zone entre la pompe sanguine et l'unité de traitement du sang et **en ce qu'**au moins la valeur de l'amplitude de pression de ces impulsions de pression est donnée par une unité d'évaluation reliée à celui-ci, des valeurs limites pouvant être prescrites dans l'unité d'évaluation et un avertissement pouvant être affiché lorsque lesdites valeurs limites sont dépassées par une valeur inférieure ou supérieure.

Figur 1

Figur 2

| Ra | Rf | Rv | PF(a) | PF(v) |
|----|----|-----|-------|-------|
| → | → | ↗ | ↗ | ↗ |
| ↗ | → | → | ↘ | ↘ |
| → | ↗ | → | ↗ | ↘ |

Figur 3

Figur 4

Figur 5

Figur 6

Arterial bloodpulse amplitude versus access pressure

Figur 7

Figur 8

**903**

Figur 9

Figur 10

500

502

503

504

501

Figur 11

**IN DER BESCHREIBUNG AUFGEFÜHRTE DOKUMENTE**

**In der Beschreibung aufgeführte Patentdokumente**

- US 5685989 A
- DE 3806248
- WO 9710013 A1
- US 4174367 A
- DE 9400924
- DE 4106444

**In der Beschreibung aufgeführte Nicht-Patentliteratur**

- **SCHWAB SJ ; RAYMOND JR ; SAEED M ; NEWMAN GE ; DENNIS PA ; BOLLINGER RR.** Prevention of hemodialysis fistula thrombosis. Early detection of venous stenoses. *Kidney International,* 1989, vol. 36, 707-11
- **BESARAB A ; AL-SAGHIR F ; ALNABHAN N ; LUBKOWSKI T ; FRINAK S.** Simplified Measurement of Intra-Access Pressure. *ASAIO Journal,* 1996, vol. 42, M682-7
- **BESARAB A ; FRINAK S.** The prevention of access failure: pressure monitoring. *ASAIO Journal,* 1998, vol. 44, 35-7